# EUROPEAN PATENT SPECIFICATION

(11) **EP 1 536 762 B1**
(45) Date of publication and mention of the grant of the patent: **17.01.2007**
(21) Application number: 03756797.1
(22) Date of filing: 10.09.2003
(51) Int. Cl.: A61K 8/89, A61Q 5/00, A61Q 5/02, A61Q 13/00

(54) **COMPOSITIONS COMPRISING SILICONE-IN-WATER EMULSIONS AND FRAGRANCES AND HAIR CARE PREPARATIONS COMPRISING SUCH COMPOSITIONS**
ZUSAMMENSETZUNGEN ENTHALTEND SILIKONE-IN-WASSER EMULSIONEN UND DUFTSTOFFE UND HAARPFLEGEZUSAMMENSETZUNGEN ENTHALTEND SOLCHE ZUSAMMENSETZUNGEN
COMPOSITIONS COMPORTANT DES EMULSIONS AQUEUSES DE SILICONE ET PRODUITS PARFUMES ET DE SOINS CAPILLAIRES COMPRENANT DE TELLES COMPOSITIONS

(30) Priority: 10.09.2002 US 409393 P
(43) Date of publication of application: 08.06.2005
(73) Proprietor: Takasago International Corporation, Tokyo 144-8721 (JP); DOW CORNING S.A., 7180 Seneffe (BE)
(72) Inventor: GIRBOUX, Anne-Lise, 1420 Braine l'Alleud (BE); MARTEAUX, Leon, 1160 Brussels (BE); NEWTON, Joanne, 1332 Geneval (BE); POLSTER-RENARD, Catherine, 7830 Hellebecq (BE); REETH, Isabelle Van, 1315 Incourt (BE); BARCKLEY, Kathleen A., Livingston, NJ 07039 (US); LUPO, Andrew T., Emerson, NJ 07630 (US); SANSONE, Robert P., Tuxedo Park, NY 10987 (US)
(74) Representative: W.P. Thompson & Co.
(86) International application number: PCT/US2003/028182
(87) International publication number: WO 2004/024114

(56) References cited:
- EP-A- 0 874 017
- WO-A-01/58986
- WO-A-02/062311
- US-A1- 2002 034 490
- XP002232355
- XP002232354

## Description

### FIELD OF THE INVENTION

The present invention relates to compositions comprising silicone-in-water emulsions and fragrance compositions. It also relates to hair care preparations comprising such compositions, which provide improved release of fragrance when applied to the hair and improved deposition of the fragrance on the hair. The invention is further related to methods of cleansing hair, which comprises applying a hair care preparation to the hair.

### BACKGROUND OF THE INVENTION

Silicone-in-water emulsions are well known for use in cosmetic preparations. Hair care preparations with silicone-in-water emulsions have been described, for example, in WO200203936, EP1093809, and WO200139735. Silicone is lubricious and acts to enhance the properties of the hair by improving the feel, the shine, and the ease of combing the hair. These compositions may also include fragrances, which are intended to be released during the application of the composition and, preferably, be deposited onto the hair for subsequent release. There is, however, still a need for hair care preparations that can provide improved deposition of fragrances or at least a deposition that will result in a longer lasting release of fragrance from the hair, without losing the favorable sensory and aesthetic performance qualities of the product.

It has now surprisingly been found that if an emulsion of a silicone polymer of certain minimum viscosity is introduced in combination with certain fragrances in the cosmetic preparation, better deposition of the fragrance can be achieved without any loss of the essential properties of hair care preparations. The better deposition particularly occurs upon joint introduction of the silicone polymer and the fragrance composition in a single emulsion composition.

WO 02/062311 A (combined with EP-A-0 874 017 internally referred to) and WO 01/58986 both disclose compositions containing a fragrance and a S/W-emulsion comprising a surfactant, wherein the silicone has a viscosity of 100 millions mm²/s or more and a particle size of 2 microns or less.

### SUMMARY OF THE INVENTION

There is provided a composition comprising: i) a fragrance composition, wherein the fragrance composition comprises a) at least 10.0% by weight of a fragrance composition of one or more perfume ingredients having a boiling point of 250 °C or less and ClogP of less than 3.0; b) at least 15% by weight of a fragrance composition of a one or more perfume ingredients having a boiling point of 250 °C or less and ClogP of 3.0 or more; and c) at least 20% by weight of a fragrance composition of one or more perfume ingredients have a boiling point of greater than 250 °C; and ii) a silicone-in-water emulsion comprising at least one surfactant wherein the dispersed silicone has a viscosity of 100 million mm²s⁻¹ or more and a particle size of less than 2 µm.

The invention is directed, in an aspect, to a method of cleansing hair, which comprises applying to the hair a hair care preparation comprising: the above composition.

### DETAILED DESCRIPTION OF THE INVENTION

### Silicone-in-water emulsions

The silicone-in-water emulsion used in the present invention may be prepared by emulsion polymerisation methods well-known in the art, such as those described in U.S. 5,998,537 and EP 0874017. In summary, this method comprises a) mixing materials comprising a composition containing at least one polysiloxane, at least one organosilicon material that reacts with the polysiloxane by a chain extension reaction, a metal containing catalyst for said chain extension reaction, at least one surfactant, and water to form a mixture, and b) emulsifying the mixture. This method provides a route to silicone-in-water emulsions having a wide range of physical characteristics, in particular emulsions wherein the dispersed silicone has a high viscosity and low droplet size.

Chain extension reactions with polysiloxanes are known in the art and can involve, for instance, the hydrosilylation reaction in which an Si-H reacts with an aliphatically unsaturated group in the presence of a platinum or rhodium-containing catalyst forming polysiloxanes having some Si-(C)ₚ-Si links (wherein p = 1-6), which polysiloxanes may also be referred to as polysiloxane polysilalkylene copolymers. Alternatively, the chain extension reaction can involve the reaction of an Si-OH group (e.g., a hydroxy-terminated polysiloxane) with an alkoxy group (e.g., alkoxysilanes, silicates, or alkoxysiloxanes) in the presence of a metal containing catalyst forming polysiloxane materials.

The polysiloxanes used in the chain extension reactions described above generally comprise a substantially linear polymer of the structure (I): 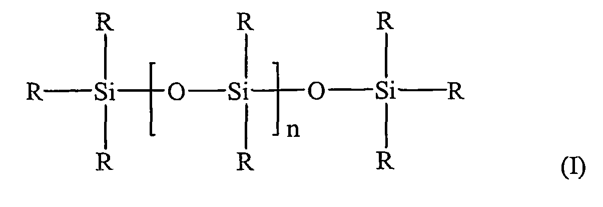 In this structure, each R independently represents a hydrocarbon group having up to 20 carbon atoms, preferably 1 to 6 carbon atoms, such as an alkyl group (e.g., methyl, ethyl, propyl or butyl), an aryl group (e.g., phenyl), or the group required for the chain extension reaction described above ("reactive group", e.g., silicon-bonded hydrogen atoms, aliphatically unsaturated groups such as vinyl, allyl or hexenyl, hydroxy, alkoxy such as methoxy, ethoxy or propoxy, alkoxy-alkoxy, acetoxy, amino and the like), provided that on average there is from one to two reactive groups (inclusive) per polymer, and *n* is a positive integer greater than one. Preferably, a majority, more preferably >90%, and most preferably >98% of the reactive groups are substituted on the terminal silicone atom in the siloxane. Preferably, n is an integer which results in polysiloxanes with viscosities between about 1 and about 1 million mm²s⁻¹, preferably from 1000 to 100,000 mm²s⁻¹.

The polysiloxane (I) can have a small amount of branching (e.g., less than 2 mole % of the siloxane units), but preferably, wherein the polymers are substantially linear, more preferably completely linear. Moreover, the R groups can be substituted with, for example, nitrogen containing groups (e.g., amino groups), epoxy groups, sulfur-containing groups, silicon-containing groups, oxygen-containing groups and the like. Preferably, however, at least 80% of the R groups are alkyl and, more preferably, the alkyl groups are methyl groups.

The organosilicon material that reacts with the polysiloxane by a chain extension reaction can be either a second polysiloxane or a material which acts as a chain extension agent. If the organosilicon material is a polysiloxane, then it too will generally have the structure described above (I). In such a situation, one polysiloxane in the reaction will comprise one reactive group and the second polysiloxane will comprise a second reactive group that reacts with the first.

If the organosilicon material comprises a chain extension agent, it can be a material such as a silane, a siloxane (e.g., disiloxane or trisiloxane) or a silazane. For example, a composition comprising a polysiloxane according to the above structure (I) which has at least one Si-OH group can be chain extended by using an alkoxysilane (e.g., a dialkoxysilane or trialkoxysilane) in the presence of a tin or titanium containing catalyst.

The metal containing catalysts used in the above described chain extension reactions are often specific to the particular reaction. Such catalysts, however, are known in the art. Generally, they are materials containing metals such as platinum, rhodium, tin, titanium, copper, lead, etc.

In a preferred chain-extension reaction, a polysiloxane having at least one aliphatically unsaturated group, preferably an end-group, is reacted with an organosilicon material that is a siloxane or a polysiloxane having at least one Si-H group, preferably an end group, in the presence of a hydrosilylation catalyst. The polysiloxane having at least one aliphatically unsaturated group has the structure (I) wherein R and n are as defined above and provided that on average between one and two (inclusive) R groups comprise an aliphatically unsaturated group per polymer. Representative aliphatically unsaturated groups include vinyl, allyl, hexenyl and cyclohexenyl or a group R²CH=CHR³, where R² represents a divalent aliphatic chain linked to the silicon atom and R³ represents a hydrogen atom or an alkyl group. The organosilicon material having at least one Si-H group preferably has the above structure (I) wherein R and *n* are as defined above and provided that on average between one and two (inclusive) R groups comprise hydrogen atoms and *n* is 0 or a positive integer. This material can be a polymer or a lower molecular weight material such as a siloxane (e.g. a disiloxane or a trisiloxane).

The polysiloxane having at least one aliphatically unsaturated group and the organosilicon material having at least one Si-H group react in the presence of a hydrosilylation catalyst. Such catalysts are known in the art and include, for example, platinum and rhodium-containing materials. These catalysts may take any of the known forms, such as platinum or rhodium deposited on carriers such as silica gel or powdered charcoal, or other appropriate compounds such as platinic chloride, salts of platinum and chloroplatinic acids. A preferred material is chloroplatinic acid either as the commonly obtainable hexahydrate or the anhydrous form because of its easy dispersibility in organosilicon systems and its non-effect on colour of the mixture. Platinum or rhodium complexes may also be used, for example those prepared from chloroplatinic acid hexahydrate and divinyltetramethyldisiloxane. Generally, these catalysts are used in amounts of between about 0.0001 and 10 wt. % based on the weight of the composition (I).

In a second preferred chain extension reaction, a polysiloxane having at least one Si-OH group, preferably an end group, is reacted with an organosilicon material having at least one alkoxy group, preferably a siloxane having at least one Si-OR group, or an alkoxysilane having at least two alkoxy groups in the presence of a metal containing catalyst. In this case, the polysiloxane having at least one SiOH group has the structure (I) wherein R and n are as defined above and on average between one and two (inclusive) R groups comprise a hydroxyl group (OH). The organosilicon material having at least one alkoxy group can have the structure (I) wherein R and n are as defined above and on average between one and two (inclusive) R groups comprise alkoxy groups, e.g., of the structure (OR) in which R is as defined above and n is 0 or a positive integer. Alternatively, the organosilicon material can be a silane of the structure RₘSi(OR)₄₋ₘ, wherein R is as defined above and *m* is 0 to 2. Other materials containing the alkoxy group (e.g., alkoxy- alkoxy) may also be used.

A variety of metal catalysts for the reaction of an Si-OH group with an Si-OR group are known in the art and may be employed including, for example, organic metal compounds such as organotin salts, titanates, or titanium chelates or complexes. Examples include stannous octoate, dibutyltin dilaurate, dibutyltin diacetate, dimethyltin dineodecanoate, dibutyltin dimethoxide, isobutyl tin triceroate, dimethyltin dibutyrate, dimethyltin dineodecanoate, triethyltin tartrate, tin oleate, tin naphthenate, tin butyrate, tin acetate, tin benzoate, tin sebacate, tin succinate, tetrabutyl titanate, tetraisopropyl titante, tetraphenyl titante, tetraoctadecyl titanate, titanium naphthanate, ethyltriethanolamine titante, titanium diiso-propyl diethyl acetoacetate, titanium diisopropoxy diacetyl acetonate, and titanium tetra alkoxides where the alkoxide is butoxy or propoxy. Generally, these catalysts are used in amounts of between about 0.001 and 10 wt. % based on the weight of the composition (I).

Any composition containing at least one polysiloxane, at least one organosilicon material which reacts with said polysiloxane by a chain extension reaction, and a metal containing catalyst for said chain extension reaction can be used for the chain extension reaction.

The dispersed silicone in the emulsion used in the present invention has a viscosity of 100 million mm²s⁻¹ or more, more preferably 150 million mm²s⁻¹ or more, and most preferably 170 million mm²s⁻¹ or more, and a particle size of 2 µm ore less, more preferably 1 µm or less, and most preferably 0.5 µm or less. (Viscosities are measured at 25°C and atmospheric pressure.) It is also a preferred option that the particle size is small enough to cause the emulsion to be a microemulsion.

The emulsion preferably comprises at least 50% wt. silicone, more preferably at least 60 wt. %, even more preferably 75 wt. %. In addition to the polymerised silicone, the emulsion may also contain up to 2.5% by weight, based on the total weight of silicone, of a volatile silicone, which is mainly selected from cyclic siloxanes, preferably dimethyl cyclo polysiloxanes, and short chain polysiloxanes, preferably polydimethyl siloxanes with a chain length of no more than 10 siloxane units.

The preferred silicone-in-water emulsions of the invention are non-ionic and the silicone is a linear polysiloxane polysilalkylene copolymer, wherein the monovalent silicone substituents are substantially all C₁₋₆ hydrocarbon groups and the copolymer has a viscosity of 150 million mm²s⁻¹ or more and a particle size of 1 µm or less. In a more preferred embodiment, the silicone-in-water emulsion is a non-ionic emulsion comprising about 60 % by weight of a linear silicone copolymer formed by the hydrosilylation of a Si-H end-blocked polydimethyl siloxane and a vinyl-end-blocked polydimethyl siloxane, wherein the copolymer has a viscosity of about 170 mm²s⁻¹ and a particle size of about 0.5 µm, and about 5 % by weight of non-ionic surfactants.

### Surfactants

The mixture used to form the emulsion also contains at least one surfactant. This can be a non-ionic surfactant, a cationic surfactant, an anionic surfactant, alkylpolysaccharides, amphoteric surfactants and the like.

Examples of non-ionic surfactants include polyoxyalkylene alkyl ethers, polyoxyalkeylene sorbitan alkyl esters, polyoxyalkylene alkyl esters, and polyoxyalkylene alkylphenol ethers, polyethylene glycols, polypropylene glycols, and diethylene glycols. Particular examples include BRIJ35 and BRIJ30 (ICI Chemicals).

Examples of cationic surfactants include quaternary ammonium hydroxides such as tetramethylammonium hydroxide, octyltrimethylammonium hydroxide, dodecyl-trimethyl ammonium hydroxide, hexadecyltrimethyl ammonium hydroxide, octyldimethylbenzylammonium hydroxide, decyldimethylbenzyl ammonium hydroxide, didodecyldimethyl ammonium hydroxide, dioctadecyl dimethylammonium hydroxide, tallow trimethylammonium hydroxide and cocotrimethylammonium hydroxide as well as corresponding salts of these materials, fatty acid amines and amides and their derivatives and the salts of the fatty acid amines and amides including aliphatic fatty amines and their derivatives, homologs of aromatic amines having fatty chains, fatty amides derived from aliphatic diamines, fatty amides derived from disubstituted amines, derivatives of ethylene diamine, amide derivatives of amino alcohols, amine salts of long chain fatty acids, quaternary ammonium bases derived from fatty amides of disubstituted diamines quaternary ammonium bases of benzimidazolines, basic compounds of pyridinium and its derivatives, sulfonium compounds, quaternary ammonium compounds of betaine, urethanes of ethylene diamine, polyethylene diamines and polypropanolpolyethanol amines.

Examples of suitable anionic surfactants include alkyl sulfates such as lauryl sulfate, polymers such as acrylates/C₁₀₋₃₀ alkyl acrylate crosspolymer alkylbenzenesulfonic acids and salts such as hexylbenzenesulfonic acid, octylbenzenesulfonic acid, decylbenzenesulfonic acid, dodecylbenzenesulfonic acid, cetylbenzenesulfonic acid and myristylbenzenesulfonic acid; the sulfate esters of monoalkyl polyoxyethylene ethers; alkylnapthylsulfonic acid; alkali metal sulforecinates, sulfonated glyceryl esters of fatty acids such as sulfonated monoglycerides of coconut oil acids, salts of sulfonated monovalent alcohol esters, amides of amino sulfonic acids, sulfonated products of fatty acid nitriles, sulfonated aromatic hydrocarbons, condensation products of naphthalene sulfonic acids with formaldehyde, sodium octahydroanthracene sulfonate, alkali metal alkyl sulfates, ester sulfates, and alkarylsulfonates.

Examples of suitable alkylpolysaccharides include, for example, materials of the structure R¹-O-(R²O)ₓ-(G)_{y} wherein R¹ represents a linear or branched alkyl group, a linear or branched alkenyl group or an alkylphenyl group, R² represent an alkylene group, G represents a reduced sugar, x denotes 0 or a positive integer and y denotes a positive integer as described, for example, in US Patent 5,035,832, incorporated herein by reference.

Examples of suitable amphoteric surfactants include cocamidopropyl betaine and cocamidopropyl hydroxysulfate.

The above surfactants may be used individually or in combination.

### Fragrance compositions

The fragrances of the present invention comprise a) at least 10.0% by weight of the fragrance composition of one or more perfume ingredients having a boiling point of 250 °C or less and ClogP of less than 3.0; b) at least 15% by weight of the fragrance composition of one or more perfume ingredients having a boiling point of 250 °C or less and ClogP of 3.0 or more, and c) at least 20% by weight of the fragrance composition of one or more perfume ingredients have a boiling point of greater than 250 °C. Examples of these three classes of perfume ingredients are illustrated in Tables 1-3. The specific ingredients in these Tables are illustrative only and other materials familiar to those skilled in the art that fall within the guidelines above can also be incorporated into the fragrance compositions of the present invention.

The fragrance compositions of the invention preferably comprise at least 20.0% by weight of the fragrance composition of one or more perfume ingredients having a boiling point of 250 °C or less and ClogP of less than 3.0; at least 15% by weight of the fragrance composition of a one or more perfume ingredients having a boiling point of 250 °C or less and ClogP of 3.0 or more; and at least 30% by weight of the fragrance composition of one or more perfume ingredients have a boiling point of greater than 250 °C. The fragrances compositions of the invention more preferably comprise: a) 30.0 to 40.0 % by weight of the fragrance composition of one or more perfume ingredients having a boiling point of 250 °C or less and ClogP of less than 3.0; b) 20.0 to 25.0 % by weight of the fragrance composition of one or more perfume ingredients having a boiling point of 250 °C or less and ClogP of 3.0 or more; and c) 40.0 to 45.0 % by weight of the fragrance composition of one or more perfume ingredients have a boiling point of greater than 250 °C.

The fragrance compositions of the invention include as examples, but are not limited to fragrance 890656 and fragrance 862883. Fragrance 890656 comprises a) 31.03 % by weight of the fragrance composition of one or more perfume ingredients having a boiling point of 250 °C or less and ClogP of less than 3.0; b) 20.17 % by weight of the fragrance composition of a one or more perfume ingredients having a boiling point of 250 °C or less and ClogP of 3.0 or more; and c) 44.91 % by weight of the fragrance composition of one or more perfume ingredients have a boiling point of greater than 250 °C. Fragrance 890656 comprises 12.79 % Di propylene glycol; 12.00 % Linalyl acetate; 9.00 % Hexyl cinnamic aldehyde; 7.50 % Tonalide; 7.38 % Benzyl salicylate; 6.05% Phenyl ethyl alcohol; 4.89 % Linalool; 4.50 % Vertenex; 10.01 % apple base; 4.93 % ozonic base; 9.84 % woody base; 10.61 % floral base; and .50 % oils or resins. Apple base, ozonic base, woody base, and floral base are fragrances comprising one or more perfume ingredients that have the combined indicated characteristic. Fragrance 890656 has the fragrance description of citrus, green, lemon, melon, apple, woody, jasmin, herbal, peach, muguet, and musk.

Fragrance 862883 comprises a) 34.08 % by weight of the fragrance composition of one or more perfume ingredients having a boiling point of 250 °C or less and ClogP of less than 3.0; b) 23.10 % by weight of the fragrance composition of a one or more perfume ingredients having a boiling point of 250 °C or less and ClogP of 3.0 or more; and c) 42.46 % by weight of the fragrance composition of one or more perfume ingredients have a boiling point of greater than 250 °C. Fragrance 862883 comprises 18.98 % Di propylene glycol; 7.35 Verdox; 4.48 % Hedion; 4.47 % Linalool; 4.30 % Benzyl salicylate; 4.23 % Methyl hydrocinnamic aldehyde; 4.06 % Dihydro myrcenol; 4.25 % Citrus oil; 3.56 % Benzyl acetate; 3.27 % Hexyl cinnamic aldehyde; 2.04 % Benzyl Benzoate; 1.36 % Linalyl acetate; 18.12 % apple base; 5.07 % floral sandelwood base; 4.91 % fruit base; 5.77 % green base; and 3.78 % oils or resins. Apple base, floral sandelwood base, fruit base, and green base are fragrances comprising one or more perfume ingredients that have the combined indicated characteristic. Fragrance 862883 has the fragrance description of apple blossom, fruity, apple, floral, sandalwood, and musk.

The boiling points (BP) of many perfume ingredients, at standard 760 mmHg are given in, e.g., "Perfume and Flavor Chemicals (Aroma Chemicals)," Steffen Arctander, published by the author, 1969. The octanol/water partition coefficient of a fragrance ingredient is the ratio between its equilibrium concentrations in octanol and in water. The partition coefficients of the preferred perfume ingredients of the present invention may be more conveniently given in the form of their logarithm to the base 10, logP. The logP values of many perfume ingredients have been reported; for example, the Pomona92 database, available from Daylight Chemical Information Systems, Inc. (Daylight CIS), Irvine, Calif., contains many, along with citations to the original literature. However, the logP values are most conveniently calculated by the "CLOGP" program, also available from Daylight CIS. This program also lists experimental logP values when they are available in the Pomona database. The "calculated logP" (ClogP) is determined by the fragment approach of Hansch and Leo (cf., A. Leo, in Comprehensive Medicinal Chemistry, Vol. 4, C. Hansch, P. G. Sammens, J. B. Taylor and C. A. Ramsden, Eds., p. 295, Pergamon Press, 1990. The fragment approach is based on the chemical structure of each perfume ingredient, and takes into account the numbers and types of atoms, the atom connectivity, and chemical bonding. The ClogP values, which represent the octanol/water partition coefficient of a fragrance ingredient, are the most reliable and widely used estimates for this physicochemical property and are preferably used instead of the experimental logP values in the selection of perfume ingredients which are useful in the present invention.

**TABLE 1. Perfume materials with boiling point less than or equal to 250 °C and CLogP less than 3.0**

| **Name** | **BP** | **CLogP** |
|---|---|---|
| Allyl Caproate | 185 | 2.772 |
| Amyl Acetate | 142 | 2.258 |
| Amyl Propionate | 161 | 2.657 |
| Anisic Aldehyde | 248 | 1.779 |
| Anisole | 154 | 2.061 |
| Benzaldehyde | 179 | 1.480 |
| Benzyl Acetate | 215 | 1.960 |
| Benzyl Acetone | 235 | 1.739 |
| Benzyl Alcohol | 205 | 1.100 |
| Benzyl Formate | 202 | 1.414 |
| Benzyl Iso Valerate | 246 | 2.887 |
| Benzyl Propionate | 222 | 2.489 |
| Beta Gamma Hexenol | 157 | 1.337 |
| Camphor Gum | 208 | 2.117 |
| laevo-Carveol | 227 | 2.265 |
| d-Carvone | 231 | 2.010 |
| laevo-Carvone | 230 | 2.203 |
| Cinnamyl Formate | 250 | 1.908 |
| cis-Jasmone | 248 | 2.712 |
| cis-3-Hexenyl Acetate | 169 | 2.243 |
| Cuminic alcohol | 248 | 2.531 |
| Cuminic aldehyde | 236 | 2.780 |
| Cyclal C | 180 | 2.301 |
| Dimethyl Benzyl Carbinol | 215 | 1.891 |
| Dimethyl Benzyl Carbinyl Acetate | 250 | 2.797 |
| Ethyl Acetate | 77 | 0.730 |
| Ethyl Aceto Acetate | 181 | 0.333 |
| Ethyl Amyl Ketone | 167 | 2.307 |
| Ethyl Benzoate | 212 | 2.640 |
| Ethyl Butyrate | 121 | 1.729 |
| Ethyl Hexyl Ketone | 190 | 2.916 |
| Ethyl-2-methyl butyrate | 131 | 2.100 |
| Ethyl Methyl Pentanoate | 143 | 2.700 |
| Ethyl Phenyl Acetate | 229 | 2.489 |
| Eucalyptol | 176 | 2.756 |
| Fenchyl Alcohol | 200 | 2.579 |
| Flor Acetate (tricyclo Decenyl Acetate) | 175 | 2.357 |
| Frutene (tricyclo Decenyl Propionate) | 200 | 2.260 |
| Geraniol | 230 | 2.649 |
| Hexenol | 159 | 1.397 |
| Hexenyl Acetate | 168 | 2.343 |
| Hexyl Acetate | 172 | 2.787 |
| Hexyl Formate | 155 | 2.381 |
| Hydratropic Alcohol | 219 | 1.582 |
| Hydroxycitronellal | 241 | 1.541 |
| Isoamyl Alcohol | 132 | 1.222 |
| Isomenthone | 210 | 2.831 |
| Isopulegyl Acetate | 239 | 2.100 |
| Isoquinoline | 243 | 2.080 |
| Ligustral | 177 | 2.301 |
| Linalool | 198 | 2.429 |
| Linalool Oxide | 188 | 1.575 |
| Linalyl Formate | 202 | 2.929 |
| Menthone | 207 | 2.650 |
| Methyl Acetophenone | 228 | 2.080 |
| Methyl Amyl Ketone | 152 | 1.848 |
| Methyl Anthranilate | 237 | 2.024 |
| Methyl Benzoate | 200 | 2.111 |
| Methyl Benzyl Acetate | 213 | 2.300 |
| Methyl Eugenol | 249 | 2.783 |
| Methyl Heptenone | 174 | 1.703 |
| Methyl Heptine Carbonate | 217 | 2.528 |
| Methyl Heptyl Ketone | 194 | 1.823 |
| Methyl Hexyl Ketone | 173 | 2.377 |
| Methyl Phenyl Carbinyl Acetate | 214 | 2.269 |
| Methyl Salicylate | 223 | 1.960 |
| Nerol | 227 | 2.649 |
| Octalactone | 230 | 2.203 |
| Octyl Alcohol (Octanol-2) | 179 | 2.719 |
| para-Cresol | 202 | 1.000 |
| para-Cresyl Methyl Ether | 176 | 2.560 |
| para-Methyl Acetophenone | 228 | 2.080 |
| Phenoxy Ethanol | 245 | 1.188 |
| Phenyl Acetaldehyde | 195 | 1.780 |
| Phenyl Ethyl Acetate | 232 | 2.129 |
| Phenyl Ethyl Alcohol | 220 | 1.183 |
| Phenyl Ethyl Dimethyl Carbinol | 238 | 2.420 |
| Prenyl Acetate | 155 | 1.684 |
| Propyl Butyrate | 143 | 2.210 |
| Pulegone | 224 | 2.350 |
| Rose Oxide | 182 | 2.896 |
| Safrole | 234 | 1.870 |
| 4-Terpinenol | 212 | 2.749 |
| alpha-Terpineol | 219 | 2.569 |
| Viridine | 221 | 1.293 |

**TABLE 2. Perfume materials with boiling point less than or equal to 250 °C and CLogP greater than or equal to 3.0**

| **Name** | **BP** | **CLogP** |
|---|---|---|
| Zallo-Ocimene | 192 | 4.362 |
| Allyl Heptoate | 210 | 3.301 |
| Anethol | 236 | 3.314 |
| Benzyl Butyrate | 240 | 3.698 |
| Camphene | 159 | 4.192 |
| Carvacrol | 238 | 3.401 |
| cis-3-Hexenyl Tiglate | 101 | 3.700 |
| Citral (Neral) | 228 | 3.120 |
| Citronellol | 225 | 3.193 |
| Citronellyl Acetate | 229 | 3.670 |
| Citronellyl Isobutyrate | 249 | 4.937 |
| Citronellyl Nitrile | 225 | 3.094 |
| Citronellyl Propionate | 242 | 4.628 |
| Cyclohexyl Ethyl Acetate | 187 | 3.321 |
| Decyl Aldehyde | 209 | 4.008 |
| Delta Damascone | 242 | 3.600 |
| Dihydro Myrcenol | 208 | 3.030 |
| Dihydromyrcenyl Acetate | 225 | 3.879 |
| Dimethyl Octanol | 213 | 3.737 |
| Fenchyl Acetate | 220 | 3.485 |
| gamma Methyl Ionone | 230 | 4.089 |
| gamma-Nonalactone | 243 | 3.140 |
| Geranyl Acetate | 245 | 3.715 |
| Geranyl Formate | 216 | 3.269 |
| Geranyl Isobutyrate | 245 | 4.393 |
| Oeranyl Nitrile | 222 | 3.139 |
| Hexenyl Isobutyrate | 182 | 3.181 |
| Hexyl Neopentanoate | 224 | 4.374 |
| Hexyl Tiglate | 231 | 3.800 |
| alpha-Ionone | 237 | 3.381 |
| beta-Ionone | 239 | 3.960 |
| gamma-Ionone | 240 | 3.780 |
| alpha-Irone | 250 | 3.820 |
| Isobornyl Acetate | 227 | 3.485 |
| Isobutyl Benzoate | 242 | 3.028 |
| Isononyl Acetate | 200 | 3.984 |
| Isononyl Alcohol | 194 | 3.078 |
| Isomenthol | 219 | 3.030 |
| para-Isopropyl Phenylacetaldehyde | 243 | 3.211 |
| Isopulegol | 212 | 3.330 |
| Lauric Aldehyde (Dodecanal) | 249 | 5.066 |
| d-Limonene | 177 | 4.232 |
| Linalyl Acetate | 220 | 3.500 |
| Menthyl Acetate | 227 | 3.210 |
| Methyl Chavicol | 216 | 3.074 |
| alpha-iso "gamma" Methyl Ionone | 230 | 4.209 |
| Methyl Nonyl Acetaldehyde | 232 | 4.846 |
| Methyl Octyl Acetaldehyde | 228 | 4.317 |
| Myrcene | 167 | 4.272 |
| Neral | 228 | 3.320 |
| Neryl Acetate | 231 | 3.555 |
| Nonyl Acetate | 212 | 4.374 |
| Nonyl Aldehyde | 212 | 3.479 |
| Octyl Aldehyde | 223 | 3.845 |
| Orange Terpenes (d-Limonene) | 177 | 4.232 |
| para-Cymene | 179 | 4.068 |
| Phenyl Ethyl Isobutyrate | 250 | 3.000 |
| alpha-Pinene | 157 | 4.122 |
| beta-Pinene | 166 | 4.182 |
| alpha-Terpinene | 176 | 4.412 |
| gamma-Terpinene | 183 | 4.232 |
| Terpinolene | 184 | 4.232 |
| Terpinyl acetate | 220 | 3.475 |
| Tetrahydro Linalool | 191 | 3.517 |
| Tetrahydro Myrcenol | 208 | 3.517 |
| Undecenal | 223 | 4.053 |
| Veratrol | 206 | 3.140 |
| Verdox | 221 | 4.059 |
| Vertenex | 232 | 4.060 |

**TABLE 3. Perfume materials with boiling point greater than 250 °C**

| **Name** | **BP** | **CLogP** |
|---|---|---|
| Allyl Cyclohexane Propionate | 267 | 3.935 |
| Ambrettolide | 300 | 6.261 |
| Amyl Benzoate | 262 | 3.417 |
| Amyl Cinnamate | 310 | 3.771 |
| Amyl Cinnamic Aldehyde | 285 | 4.324 |
| Amyl Cinnamic Aldehyde Dimethyl Acetal | 300 | 4.033 |
| iso-Amyl Salicylate | 277 | 4.601 |
| Aurantiol | 450 | 4.216 |
| Benzophenone | 306 | 3.120 |
| Benzyl Salicylate | 300 | 4.383 |
| Cadinene | 275 | 7.346 |
| Cedrol | 291 | 4.530 |
| Cedryl Acetate | 303 | 5.436 |
| Cinnamyl Cinnamate | 370 | 5.480 |
| Coumarin | 291 | 1.412 |
| Cyclohexyl Salicylate | 304 | 5.265 |
| Cyclamen Aldehyde | 270 | 3.680 |
| Dihydro Isojasmonate | 300 | 3.009 |
| Diphenyl Methane | 262 | 4.059 |
| Ethylene Brassylate | 332 | 4.554 |
| Ethyl Methyl Phenyl Glycidate | 260 | 3.165 |
| Ethyl Undecylenate | 264 | 4.888 |
| iso-Eugenol | 266 | 2.547 |
| Exaltolide | 280 | 5.346 |
| Galaxolide | 260 | 5.482 |
| Oeranyl Anthranilate | 312 | 4.216 |
| Hexadecanolide | 294 | 6.805 |
| Hexenyl Salicylate | 271 | 4.716 |
| Hexyl Cinnamic Aldehyde | 305 | 5.473 |
| Hexyl Salicylate | 290 | 5.260 |
| Linalyl Benzoate | 263 | 5.233 |
| 2-Methoxy Naphthalene | 275 | 3.235 |
| Methyl Cinnamate | 263 | 2.620 |
| Methyl Dihydrojasmonate | 300 | 2.275 |
| beta-Methyl Naphthyl ketone | 300 | 2.275 |
| Musk Indanone | >250 | 5.458 |
| Musk Ketone | 137 (MP) | 3.014 |
| Musk Tibetine | 136 (MP) | 3.831 |
| Myristicin | 276 | 3.200 |
| delta-Nonalactone | 280 | 2.760 |
| Oxahexadecanolide-10 | 300 | 4.336 |
| Oxahexadecanolide-11 | 35 (MP) | 4.336 |
| Patchouli Alcohol | 285 | 4.530 |
| Phantolide | 288 | 5.977 |
| Phenyl Ethyl Benzoate | 300 | 4.058 |
| Phenylethylphenylacetate | 325 | 3.767 |
| alpha-Santalol | 301 | 3.800 |
| Thibetolide | 280 | 6.246 |
| delta-Undecalactone | 290 | 3.830 |
| gamma-Undecalacto ne | 297 | 4.140 |
| Vanillin | 285 | 1.580 |
| Vetiveryl Acetate | 285 | 4.882 |
| Yara-Yara | 274 | 3.235 |

### Hair Care Preparations

The silicone-in-water emulsion, wherein the dispersed silicone has a viscosity of at least, and preferably more than 100 million mm²s⁻¹, and a particle size of at most, preferably less than 2 µm, is mixed with a fragrance composition in a weight ratio of preferably from 1:1 to 10:1 1 of silicone:fragrance. It is preferred that this mixing takes place prior to incorporation in a hair care preparation, as it enables a greater control and consistency of the distribution and partitioning of the silicone and the fragrance in the hair care preparation. Mixing may take place by methods well-known in the art. For example, low shear mixing may take place by methods well-known in the art, as for example, regular hand mixing using low shear bench scale overhead mixing equipment, as for example a Heidolf RZR 50 stirrer with variable speed range between 45-2000 RPM, or on a larger scale in a groin kettle or via large scale overhead mechanical drum mixing.

The mixture is then incorporated in a hair care preparation in an amount which provides at least 1% silicone by weight of the hair care preparation, for example 1 to 10% by weight, or for example 2 to 6% wt. Hair care preparations, including shampoos, conditioners and conditioning shampoos are well known in the art, and typically comprise a washing base containing surfactants, and other agents for improving the cosmetic properties of the preparation. The washing base typically comprises a cosmetically acceptable aqueous medium, which may be solely water, or water with a solvent (for example, a lower alcohol or alkylene glycol). The surfactants may be anionic, non-ionic, cationic, or amphoteric. Other additives which may be included in the preparation include pH adjusters (for example, amines), viscosity regulators, electrolytes or thickeners, pearlising or opacifying agents, foam synergists, and other agents for improving the properties of the preparation, for example anionic, non-ionic and cationic polymers, proteins, fatty acids and vitamins. The hair care preparation will typically be in the form of a liquid, a thickened liquid, a lotion or a gel.

The use of the above defined silicone-in-water emulsion in hair care preparations according to the present invention can provide hair care preparations which have improved fragrance deposition on hair, measured as longer lasting fragrance release from the hair compared to conventional preparations. The improved fragrance deposition occurs without a loss of favorable sensory and aesthetic perception for the user normally associated with silicone deposition on hair, which includes ease of combing, luster, shine and general conditioning.

It is particularly surprising that the combination of such high viscosity of the emulsified silicones with such low particle size results in the greater deposition of the fragrance or a longer lasting release of the fragrance from the hair, without negatively affecting the benefits of deposited silicones.

The invention is directed to a method of cleansing hair, which comprises applying to the hair a hair care preparation comprising: i) a fragrance composition; and ii) a silicone-in-water emulsion comprising at least one surfactant, wherein the dispersed silicone has a viscosity of 100 million mm²s⁻¹ or more and a particle size of less than 2 µm.

In a preferred embodiment, the silicone:fragrance composition weight ratio is from 1:1 to 10:1. In a further preferred embodiment, the hair care preparation is a shampoo, a conditioner, or a conditioning shampoo.

The compositions of the invention may additionally be used in other cosmetic applications, in particular skin care applications, providing a similar benefit. Examples of suitable skin care applications include lotions, gels (including shower gels), jellies, pastes, facial applications, moisturizers, face masks, night creams, day creams, sun milk, massage creams, make-up removal lotions, anti-aging cream, and anti-wrinkle cream.

The following Examples are provided for a further illustration of the invention, but are not intended to be limiting thereof. Unless otherwise indicated, all parts and percentages are by weight and all viscosities are dynamic viscosities measured at 25 °C.

### Examples

### Example 1

A silicone-in-water emulsion, SEM1, was made by mixing 58.5 parts dimethylvinyl-terminated polydimethylsiloxane having a viscosity of 55,000 mm²s⁻¹ with 1.17 parts of dimethylhydrogen-terminated polydimethylsiloxane having a viscosity of 10 mm²s⁻¹. To this mixture was added 2.05 parts of polyoxyethylene (3) lauryl ether and enough water to cause phase inversion. 2.8 parts of polyoxyethylene (23) lauryl ether were then added under high shear. Finally, water was added to a total of 35.5 parts water, in which the water was added in stages with mixing after each addition, followed by the addition of 0.012 parts of a platinum catalyst and 0.5 parts of Glydant Plus preservative. The resulting emulsion was a non-ionic emulsion containing about 60% by weight of a linear silicone polymer formed from the hydrosilylation of a Si-H end-blocked polydimethyl siloxane and a vinyl-end-blocked polydimethyl siloxane, in which the polymer has a viscosity of about 170 million mm ²s⁻¹ and an average particle size of 0.5 µm. The silicone-in-water emulsion formed, SEM1, was mixed with fragrances and tested as described in the following Examples.

### Example 2

Fragrance 862883 was mixed with the silicone-in-water emulsion, SEM1, in a 1:4 ratio by weight. The fragrance/silicone mixture was added at a concentration of 4% by weight to a shampoo base T-058.

Shampoo base T-058 is formed by mixing 0.30 % Metholcel E4M (hydroxypropyl methylcellulose; Dow Chemical Co.) in hot deionized water at 80 - 90 °C with an overhead mixer at medium speed until all of the particles are wetted down and a consistent dispersion is obtained. 0.05 % Sequestrene Na₄ (tetrasodium EDTA; Ciba Geigy) was mixed at 85 °C with deionized water, was cooled down, and added to the hot mixture. The resulting solution was cooled to 30 °C and mixing was continued for approximately 20 minutes. The mixture was reheated to 60 °C. 18.00 % Standapol T (TEA lauryl sulfate; Cognis/Henkel), 8.00 % Standapol A (ammonium lauryl sulfate; Cognis/Henkel), 4.00 % Monamid 150-LMWC (lauramide DEA; Uniqema), and 0.30 % Palmitic acid (Ruger) were added while mixing at low/medium speed using an overhead mixer. 0.15 % Gydant (DMDM hydantoin; Lonza), approximately 0.3 % sodium chloride (to adjust viscosity), and approximately 0.5 % citric acid (to adjust pH to 5.5-6.0) was added while mixing at low/medium speed using an overhead mixer. The mixture was mixed at low speed until room temperature was reached to form T-058.

As a control, fragrance alone was added at a concentration of 0.8% by weight to the same shampoo base T-058. The fragrance/silicone mixture and the control each were added to half a head of two subjects. The hair was shampooed and the fragrance was evaluated by a panel of five perfume and odor evaluators at the stages of neat products, hair during lathering, hair after rinsing, hair after blow drying, and hair 6 hours after drying. The evaluations were determined on a scale of 1-5 (1, no fragrance, to 5, strong fragrance smell) for each subject. The evaluations for the two subjects were averaged and the evaluation results are summarized in Table 4.

**TABLE 4. Effect of silicone on fragrance performance of hair care preparation.**

| **Stage** | **4% fragrance/silicone mixture in shampoo base T-058** | **0.8 % fragrance mixture in shampoo base T-058** |
|---|---|---|
| **Neat** | 4.00 | 4.25 |
| **Lathering** | 3.25 | 2.75 |
| **Rinse** | 2.75 | 1.50 |
| **Blow-dry** | 2.50 | 1.75 |
| **6 hours after blow-dry** | 2.00 | 1.50 |

The results indicate that silicones have a significant effect on residual fragrance perception at the stages of lathering, rinse, blow-dry, and 6 hours after blow-dry.

### Example 3

Fragrance 862883 was mixed with the silicone-in-water emulsion, SEM1, in a 1:4 ratio by weight. The fragrance/silicone mixture was added at a concentration of 4% by weight to a shampoo base T-058 (see Example 2). As a control, fragrance alone was added at a concentration of 0.8% by weight to the same shampoo base T-058. The fragrance/silicone mixture and the control were added to 6 swatches of hair each. The 12 hair swatches were shampooed and the fragrance was evaluated by a panel of five perfume and odor evaluators at the stages of hair after rinsing, hair after blow drying, and hair 6 hours after drying. The evaluations were determined on a scale of 1-5 (1, no fragrance, to 5, strong fragrance smell) for each swatch. The evaluations by each evaluator and for all the swatches were averaged, and the evaluation results are indicated in Table 5.

**TABLE 5. Effect of silicone on fragrance performance of hair care preparation.**

| **Stage** | **4% fragrance/silicone mixture in shampoo base T-058** | **0.8 % fragrance mixture in shampoo base T-058** |
|---|---|---|
| **Rinse** | 3.60 | 3.26 |
| **Blow-dry** | 3.02 | 2.75 |
| **6 hours after blow-dry** | 1.93 | 2.13 |

The results indicate that silicones have a positive effect on residual fragrance perception at the stages of after rinse and after blow-dry.

### Example 4

Fragrance 890656 was mixed with the silicone-in-water emulsion, SEM1, in a 1:8 ratio by weight. The fragrance/silicone mixture was added at a concentration of 3.2% by weight to a conditioner base T-114.

Conditioner base T-114 is formed by heating deionized water to 70 °C and slowing adding 0.25 % methyl paraben (TRI-K) while mixing at medium speed until completely solubilized. The resulting composition is held at 70 °C. 1.50 % propylene glycol and 1.00 % Natrosol 250 HR (hydroxythylcellulose; Hercules) are premixed into a slurry and added to the initial mixture. The resulting mixture is mixed at 70 °C until completely clear and hydrated. 1.00 % Ammonyx 4002 (stearalkonium chloride; Stephan) is slowly added to the mixture. 0.25 % Lipoquat R (ricinoleamidopropyl ethyldimonium ethosulfate; Lipo Chemicals, Inc.), 0.50 % Lipopeg 6000 DS (PEG-150 distearate; Lipo Chemicals, Inc.), 1.00 % Lipowax D (cetearyl alcohol and ceteareth-20; Lipo Chemicals, Inc.), and 0.10 % propyl paraben (TRI-K) were pre-heated until melted and clear and then added to the mixture while mixing on a homomixer at medium speed for 1 minute to form conditioner base T-114.

As a control, fragrance alone was added at a concentration of 0.4% by weight to the same conditioner base T-114. The fragrance/silicone mixture and the control were added to 6 swatches of hair each. The 12 hair swatches were conditioned and the fragrance was evaluated by a panel of five perfume and odor evaluators at the stages of hair after rinsing, hair after blow drying, and hair 6 hours after drying. The evaluations were determined on a scale of 1-5 (1, no fragrance, to 5, strong fragrance smell) for each swatch. The evaluations by each evaluator and for all the swatches were averaged and the evaluation results are indicated in Table 6.

**TABLE 6. Effect of silicone on fragrance performance of hair care preparation.**

| **Stage** | **3.2% fragrance/silicone mixture in conditioner base T-114** | **0.4 %fragrance mixture in conditioner base T-114** |
|---|---|---|
| **Rinse** | 3.23 | 2.83 |
| **Blow-dry** | 2.68 | 2.42 |
| **6 hours after blow-dry** | 2.03 | 2.00 |

The results indicate that silicones have a positive effect on residual fragrance perception at the stages of after rinse and after blow-dry.

### Example 5

Fragrance 862883 was mixed with the silicone-in-water emulsion, SEM2. SEM2 was prepared in a similar manner as SEM1 described in Example 1, except that the non-ionic surfactants were replaced with 3.4 parts of Bioterge AS 40 (Stephan Company) and less dimethylhydrogen-terminated polydimethylsiloxane was used, resulting in an anionic emulsion having a polymer with a viscosity of 300 million mm²s⁻¹ and an average particle size of 1.1 µm. The emulsion and the fragrance were mixed in a 4:1 ratio by weight. The SEM2-based fragrance/silicone mixture was added at a concentration of 4% by weight to a shampoo base T-058 (see Example 2).

Fragrance 862883 was mixed with the silicone-in-water emulsion, SEM8. SEM8 was prepared in a similar manner as SEM1 described in Example 1, except that in addition, 4 parts of a alkylmethyl siloxane polymer of about 10 mm²s⁻¹ having a C₃₀ alkyl substituent on each silicon atom, was added. The emulsion had an average particle size of 0.8 µm. The emulsion was mixed with fragrance in a 4:1 ratio by weight. The SEM8-based fragrance/silicone mixture was added at a concentration of 4% by weight to a shampoo base T-058.

As a control, fragrance alone was added at a concentration of 0.8% by weight to the same shampoo base T-058. The fragrance/silicone mixtures and the control were added to 3 swatches of hair each. The 9 hair swatches were shampooed and the fragrance was evaluated by a panel of six perfume and odor evaluators at the stages of hair after rinsing, hair after blow drying, and hair 6 hours after drying. The evaluations were determined on a scale of 1-5 (1, no fragrance, to 5, strong fragrance smell) for each swatch. The evaluations by each evaluator and for all the swatches were averaged and the evaluation results are indicated in Table 7.

**TABLE 7. Effect of silicone on fragrance performance of hair care preparation.**

| **Stage** | **4% SEM2-based fragrance/silicone mixture in shampoo base T-058** | **4% SEM8-based fragrance/silicone mixture in shampoo base T-058** | **0.4 % fragrance mixture in shampoo base T-058** |
|---|---|---|---|
| **Rinse** | 2.82 | 3.44 | 3.29 |
| **Blow-dry** | 2.00 | 2.71 | 2.74 |
| **6 hours after blow-dry** | 1.58 | 1.94 | 1.95 |

The results indicate that the SEM2 and SEM8-based mixtures do not have a consistent positive effect on residual fragrance perception.

### Example 6

Fragrance 862883 was mixed with the silicone-in-water emulsion, SEM1, in a 1;4 ratio by weight. The SEM1-based fragrance/silicone mixture was added at a concentration of 4% by weight to a shampoo base T-058 (see Example 2).

Fragrance 862883 was mixed with the silicone-in-water emulsion, SEM4, in a 1:4 ratio by weight. SEM4 was made in a similar way to SEM1 described in Example 1, however the resulting emulsion has a viscosity of 138 million mm²s⁻¹ and an average particle size of 0.35 µm. The SEM4-based fragrance/silicone mixture was added at a concentration of 4% by weight to a shampoo base T-058.

Fragrance 862883 was mixed with the silicone-in-water emulsion, SEM5, in a 1:4 ratio by weight. SEM5 was made in a similar fashion as SEM1 described in Example 1, but the resulting emulsion had a viscosity of 272 million mm²s⁻¹ and an average particle size of 0.36 µm. The SEM5-based fragrance/silicone mixture was added at a concentration of 4% by weight to a shampoo base T-058.

As a control, fragrance alone was added at a concentration of 0.8% by weight to the same shampoo base T-058. The fragrance/silicone mixtures and the control were added to 3 swatches of hair each. The 12 hair swatches were shampooed and the fragrance was evaluated by a panel of six perfume and odor evaluators at the stages of hair after rinsing, hair after blow drying, and hair 6 hours after drying. The evaluations were determined on a scale of 1-5 (1, no fragrance, to 5, strong fragrance smell) for each swatch. The evaluations by each evaluator and for all the swatches were averaged and the evaluation results are indicated in Table 8.

**TABLE 8. Effect of silicone on fragrance performance of hair care preparation.**

| **Stage** | **4% SEM1-based fragrance/silicone mixture in shampoo base T-058** | **4% SEM4-based fragrance/silicone mixture in shampoo base T-058** | **4% SEM5-based fragrance/silicone mixture in shampoo base T-058** | **0.8 % fragrance mixture in shampoo base T-058** |
|---|---|---|---|---|
| **Rinse** | 3.72 | 3.36 | 3.58 | 3.10 |
| **Blow-dry** | 3.43 | 2.97 | 3.07 | 2.92 |
| **6 hours after blow-dry** | 2.11 | 1.83 | 2.03 | 1.64 |

The results indicate that the SEM1, SEM4, and SEM5-based mixtures all give a positive effect on residual fragrance perception. Furthermore, SEM1-based mixture give a better effect than SEM4 or SEM5-based mixtures.

### Example 7

Compositions of shampoo base T-058 alone or in combination with silicone-in-water emulsion SEM1 and/or fragrance 862883 were formulated according to the ingredients and amounts given in Table 9. The viscosity of the resulting compositions are shown in Table 9. Addition of silicone-in-water emulsion SEM1 to shampoo base T-058 reduced the viscosity of the shampoo base T-058 by about 15%. Addition of fragrance 862883 increased the viscosity of shampoo base T-058 over 2.5-fold. When added to shampoo base T-058, the combination of silicone-in-water emulsion SEM1 and fragrance 862883 exhibited a viscosity reduction of about 15%, relative to addition of fragrance 862883 alone.

**TABLE 9. Effect of silicone on viscosity of shampoo-fragrance compositions**

| **Composition** | **Viscosity (cps)** |
|---|---|
| Shampoo base T-058 | 2020 |
| Shampoo base T-058 + 3.2% silicone-in-water emulsion SEM1 | 1700 |
| Shampoo base T-058+ 0.8% fragrance 862883 | 5450 |
| Shampoo base T-058 + 3.2% silicone-in-water emulsion SEM1 + 4.0% fragrance 862883 | 4530 |

The present invention is not to be limited in scope by the specific embodiments described herein. Various modifications of the invention in addition to those described herein will become apparent to those skilled in the art from the foregoing description. Such modifications are intended to fall within the scope of the appended claims.

## Claims

1. A composition comprising:
i) a fragrance composition comprising
a) at least 10.0% by weight of a fragrance composition of one or more perfume ingredients having a boiling point of 250°C or less and ClogP of less than 3.0;
b) at least 15% by weight of a fragrance composition of one or more perfume ingredients having a boiling point of 250°C or less and ClogP of 3.0 or more; and
c) at least 20% by weight of a fragrance composition of one or more perfume ingredients have a boiling point of greater than 250°C; and
ii) A silicone-in-water emulsion comprising at least one surfactant, wherein the dispersed silicone has a viscosity (dynamic viscosity measured at 25°C) of 100 million mm²s⁻¹ or more and a particle size of 2µm or less.

2. The composition of claim 1, wherein the silicone-in-water emulsion is non-ionic and the silicone is a linear polysiloxane polysilalkylene copolymer, wherein the monovalent silicone substituents are substantially all C₁₋₆ hydrocarbon groups and the copolymer has a viscosity of 150 million mm²s⁻¹ or more and a particle size of 1 µm or less.

3. The composition of claim 2, wherein the silicone-in-water emulsion is a non-ionic emulsion comprising about 60 % by weight of a linear silicone copolymer formed by the hydrosilylation of a Si-H end-blocked polydimethyl siloxane and a vinyl-end-blocked polydimethyl siloxane, wherein the copolymer has a viscosity of about 170 million mm²s⁻¹ and a particle size of about 0.5 µm, and about 5% by weight of non-ionic surfactants.

4. The composition of any one of claims 1-3, wherein said fragrance composition comprises:
a) at least 10.0% by weight of a fragrance composition of one or more perfume ingredients having a boiling point of 250 °C or less and ClogP of less than 3.0,
b) at least 20% by weight of a fragrance composition of one or more perfume ingredients having a boiling point of 250°C or less and ClogP of 3.0 or more, and
c) at least 30% by weight of a fragrance composition of one or more perfume ingredients have a boiling point of greater than 250 °C; and

5. The composition of any one of claims 1-3, wherein said fragrance composition comprises:
a) at least 20.0% by weight of the fragrance composition of one or more perfume ingredients having a boiling point of 250 °C or less and ClogP of less than 3.0;
b) at least 15% by weight of the fragrance composition of one or more perfume ingredients having a boiling point of 250 °C or less and ClogP of 3.0 or more; and
c) at least 30% by weight of the fragrance composition of one or more perfume ingredients have a boiling point of greater than 250 °C.

6. The composition of any one of claims 1-3, wherein said fragrance composition comprises:
a) 30.0 to 40.0 % by weight of the fragrance composition of one or more perfume ingredients having a boiling point of 250 °C or less and ClogP of less than 3.0;
b) 20.0 to 25.0 % by weight of the fragrance composition of a one or more perfume ingredients having a boiling point of 250 °C or less and ClogP of 3.0 or more; and
c) 40.0 to 45.0 % by weight of the fragrance composition of one or more perfume ingredients have a boiling point of greater than 250 °C.

7. The composition of any one of claims 1-6, wherein the silicone: fragrance composition weight ratio is from 1:1 to 10:1.

8. A hair care preparation comprising a composition according to any one of claims 1-8.

9. The hair care preparation of claim 8, wherein the hair care preparation is a shampoo, a conditioner, or a conditioning shampoo.

10. A method of cleansing hair, which comprises applying to the hair a hair care preparation according to claim 8 or 9.

11. A method of improving fragrance deposition on hair, which comprises applying to the hair a hair care preparation according to claim 8 or 9.

## Patentansprüche

1. Zusammensetzung, die Folgendes umfasst:
i) eine Duftstoffzusammensetzung, die Folgendes umfasst
a) mindestens 10,0 Gewichts-% einer Duftstoffzusammensetzung von einem oder mehreren Parfümbestandteilen mit einem Siedepunkt von 250 °C oder weniger und ClogP von weniger als 3,0;
b) mindestens 15 Gewichts-% einer Duftstoffzusammensetzung von einem oder mehreren Parfümbestandteilen mit einem Siedepunkt von 250 °C oder weniger und ClogP von 3,0 oder mehr; und
c) mindestens 20 Gewichts-% einer Duftstoffzusammensetzung von einem oder mehreren Parfümbestandteilen mit einem Siedepunkt von größer als 250 °C; und
ii) eine Silikon-in-Wasser-Emulsion, die mindestens einen oberflächenaktiven Stoff umfasst, worin das dispergierte Silikon eine Viskosität (dynamische Viskosität bei 25 °C gemessen) von 100 Millionen mm²s⁻¹ oder mehr und eine Partikelgröße von 2 µm oder weniger hat.

2. Zusammensetzung nach Anspruch 1, worin die Silikon-in-Wasser-Emulsion nichtionisch ist und das Silikon ein lineares Polysiloxan-Polysilalkylen-Copolymer ist, worin die monovalenten Silikon-Substituenten im Wesentlichen alle C₁₋₆-Kohlenwasserstoff-Gruppen sind und das Copolymer eine Viskosität von 150 Millionen mm²s⁻¹ oder mehr und eine Partikelgröße von 1 µm oder weniger hat.

3. Zusammensetzung nach Anspruch 2, worin die Silikon-in-Wasser-Emulsion eine nichtionische Emulsion ist, die etwa 60 Gewichts-% eines linearen Silikon-Copolymers, das durch die Hydrosilylierung eines Si-H-endblockierten Polydimethylsiloxans und eines vinyl-endblockierten Polydimethylsiloxans gebildet ist, worin das Copolymer eine Viskosität von etwa 170 Millionen mm²s⁻¹ und eine Partikelgröße von etwa 0,5 µm hat, und etwa 5 Gewichts-% von nichtionischen oberflächenaktiven Stoffen umfasst.

4. Zusammensetzung nach einem der Ansprüche 1-3, worin genannte Duftstoffzusammensetzung Folgendes umfasst:
a) mindestens 10,0 Gewichts-% einer Duftstoffzusammensetzung von einem oder mehreren Parfümbestandteilen mit einem Siedepunkt von 250 °C oder weniger und ClogP von weniger als 3,0,
b) mindestens 20 Gewichts-% einer Duftstoffzusammensetzung von einem oder mehreren Parfümbestandteilen mit einem Siedepunkt von 250 °C oder weniger und ClogP von 3,0 oder mehr, und
c) mindestens 30 Gewichts-% einer Duftstoffzusammensetzung von einem oder mehreren Parfümbestandteilen mit einem Siedepunkt von größer als 250 °C; und

5. Zusammensetzung nach einem der Ansprüche 1-3, worin genannte Duftstoffzusammensetzung Folgendes umfasst:
a) mindestens 20,0 Gewichts-% der Duftstoffzusammensetzung von einem oder mehreren Parfümbestandteilen mit einem Siedepunkt von 250 °C oder weniger und ClogP von weniger als 3,0;
b) mindestens 15 Gewichts-% der Duftstoffzusammensetzung von einem oder mehreren Parfümbestandteilen mit einem Siedepunkt von 250 °C oder weniger und ClogP von 3,0 oder mehr; und
c) mindestens 30 Gewichts-% der Duftstoffzusammensetzung von einem oder mehreren Parfümbestandteilen mit einem Siedepunkt von größer als 250 °C.

6. Zusammensetzung nach einem der Ansprüche 1-3, worin genannte Duftstoffzusammensetzung Folgendes umfasst:
a) 30,0 bis 40,0 Gewichts-% der Duftstoffzusammensetzung von einem oder mehreren Parfümbestandteilen mit einem Siedepunkt von 250 °C oder weniger und ClogP von weniger als 3,0;
b) 20,0 bis 25,0 Gewichts-% der Duftstoffzusammensetzung von einem oder mehreren Parfümbestandteilen mit einem Siedepunkt von 250 °C oder weniger und ClogP von 3,0 oder mehr; und
c) 40,0 bis 45,0 Gewichts-% der Duftstoffzusammensetzung von einem oder mehreren Parfümbestandteilen mit einem Siedepunkt von größer als 250 °C.

7. Zusammensetzung nach einem der Ansprüche 1-6, worin das Gewichtsverhältnis der Silikon:Duftstoff-Zusammensetzung von 1 : 1 bis 10 : 1 beträgt.

8. Haarpflegezubereitung, die eine Zusammensetzung nach einem der Ansprüche 1-8 umfasst.

9. Haarpflegezubereitung nach Anspruch 8, worin die Haarpflegezubereitung ein Shampoo, eine Pflegespülung oder ein Pflegeshampoo ist.

10. Verfahren zur Reinigung von Haar, das die Auftragung einer Haarpflegezubereitung nach Anspruch 8 oder 9 auf das Haar umfasst.

11. Verfahren zur Verbesserung der Duftstoffauftragung auf Haar, das die Auftragung einer Haarpflegezubereitung nach Anspruch 8 oder 9 auf das Haar umfasst.

## Revendications

1. Composition comprenant :
i) une composition de fragrance comprenant
a) au moins 10,0 % en poids d'une composition de fragrance d'un ou de plusieurs ingrédients de parfum ayant un point d'ébullition de 250°C ou moins et un ClogP inférieur à 3,0 ;
b) au moins 15 % en poids d'une composition de fragrance d'un ou de plusieurs ingrédients de parfum ayant un point d'ébullition de 250°C ou moins et un ClogP supérieur ou égal à 3,0 ; et
c) au moins 20 % en poids d'une composition de fragrance d'un ou de plusieurs ingrédients de parfum ayant un point d'ébullition supérieur à 250°C; et
ii) une émulsion silicone dans eau comprenant au moins un tensioactif, dans laquelle le silicone dispersé présente une viscosité (viscosité dynamique mesurée à 25°C) de 100 millions mm².s⁻¹ ou plus et une taille de particule de 2 µm ou moins.

2. Composition selon la revendication 1, dans laquelle l'émulsion silicone dans eau est non ionique et le silicone est un copolymère polysiloxane-polysilalkylène linéaire, où les substituants siliconés monovalents sont substantiellement tous groupes hydrocarbonés en C₁₋₆ et le copolymère présente une viscosité de 150 millions mm².s⁻¹ ou plus et une taille de particule de 1 µm ou moins.

3. Composition selon la revendication 2, dans laquelle l'émulsion silicone dans eau est une émulsion non ionique comprenant d'environ 60 % en poids d'un copolymère de silicone linéaire formé par hydrosilylation d'un polydiméthylsiloxane bloqué à une extrémité par Si-H et d'un polydiméthylsiloxane bloqué à une extrémité par un vinyle, où le copolymère possède une viscosité d'environ 170 millions mm².s⁻¹, une taille de particule d'environ 0,5 µm et environ 5 % en poids de tensioactifs non ioniques.

4. Composition selon l'une quelconque des revendications 1-3, dans laquelle ladite composition de fragrance comprend :
a) au moins 10,0 % en poids d'une composition de fragrance d'un ou de plusieurs ingrédients de parfum ayant un point d'ébullition de 250°C ou moins et un ClogP inférieur à 3,0,
b) au moins 20 % en poids d'une composition de fragrance d'un ou de plusieurs ingrédients de parfum ayant un point d'ébullition de 250°C ou moins et un ClogP supérieur ou égal à 3,0, et
c) au moins 30 % en poids d'une composition de fragrance d'un ou de plusieurs ingrédients de parfum ayant un point d'ébullition supérieur à 250°C; et

5. Composition selon l'une quelconque des revendications 1-3, dans laquelle ladite composition de fragrance comprend:
a) au moins 20,0 % en poids de la composition de fragrance d'un ou de plusieurs ingrédients de parfum ayant un point d'ébullition de 250°C ou moins et un ClogP inférieur à 3,0,
b) au moins 15 % en poids de la composition de fragrance d'un ou de plusieurs ingrédients de parfum ayant un point d'ébullition de 250°C ou moins et un ClogP supérieur ou égal à 3,0, et
c) au moins 30 % en poids de la composition de fragrance d'un ou de plusieurs ingrédients de parfum ayant un point d'ébullition supérieur à 250°C.

6. Composition selon l'une quelconque des revendications 1-3, dans laquelle ladite composition de fragrance comprend :
a) de 30,0 à 40,0 % en poids de la composition de fragrance d'un ou de plusieurs ingrédients de parfum ayant un point d'ébullition de 250°C ou moins et un ClogP inférieur à 3,0;
b) de 20,0 à 25,0 % en poids de la composition de fragrance d'un ou de plusieurs ingrédients de parfum ayant un point d'ébullition de 250°C ou moins et un ClogP supérieur ou égal à 3,0; et
c) de 40,0 à 45,0 % en poids de la composition de fragrance d'un ou de plusieurs ingrédients de parfum ayant un point d'ébullition supérieur à 250°C.

7. Composition selon l'une quelconque des revendications 1-6, dans laquelle le rapport pondéral de la composition silicone:fragrance est de 1:1 à 10:1.

8. Préparation capillaire comprenant une composition selon l'une quelconque des revendications 1-8.

9. Préparation capillaire selon la revendication 8, dans laquelle la préparation capillaire est un shampooing, un conditionneur ou un shampooing conditionneur.

10. Méthode de nettoyage des cheveux, comprenant l'application, sur les cheveux, d'une préparation capillaire selon la revendication 8 ou 9.

11. Méthode d'amélioration du dépôt de fragrance sur les cheveux comprenant l'application, sur les cheveux, d'une préparation capillaire selon la revendication 8 ou 9.
